Europäisches Patentamt

European Patent Office

Office européen des brevets

(11) Publication number: **0 267 093 B1**

# EUROPEAN PATENT SPECIFICATION

(45) Date of publication of patent specification: **18.03.92**  (51) Int. Cl.⁵: **C12M 1/22**

(21) Application number: **87402331.0**

(22) Date of filing: **19.10.87**

(54) **Container intended to receive one or more micro-organism culture mediums.**

(30) Priority: **20.10.86 FR 8614551**

(43) Date of publication of application:
**11.05.88 Bulletin 88/19**

(45) Publication of the grant of the patent:
**18.03.92 Bulletin 92/12**

(84) Designated Contracting States:
**BE CH DE GB IT LI NL SE**

(56) References cited:
**WO-A-82/00834**
**DE-B- 1 069 833**
**US-A- 4 598 050**

(73) Proprietor: **MILLIPORE S.A.**
**Zone Industrielle**
**F-67120 Molsheim(FR)**

(72) Inventor: **Lemonnier, Jean**
**63, allée de la Meute**
**F-78110 Le Vesinet(FR)**

(74) Representative: **Rinuy, Santarelli**
**14, avenue de la Grande Armée**
**F-75017 Paris(FR)**

# Description

The object of the present invention is a container intended to receive one or more culture mediums for micro-organisms, a method of filling this device with such a medium and its application to the detection of micro-organisms with a view to counting and identifying them.

In many industries, in particular the foodstuffs, pharmaceuticals and electronics industries, and in hospitals it is essential to be able to evaluate the degree to which a certain number of products or surfaces are contaminated by micro-organisms such as bacteria, yeast and mold.

To carry out this evaluation in the case of a liquid product a sample is usually filtered through a sterile filter membrane, the filter membrane is placed on a gel culture medium in a container and this container is then incubated at a temperature and for a time sufficient to enable any micro-organisms that may be present in the sample to grow and multiply in the form of colonies visible to the naked eye, so that they can be counted and identified.

One operation that is important to correct implementation of this technique for counting germs on a microporous filter membrane is the placing of the filter membrane used to separate out the micro-organisms in contact with the gel culture medium.

The surface state of the gel culture medium is therefore an important factor in the success of the operation.

There currently exist containers referred to as Petri dishes made from glass or a plastics material, usually supplied in a sterile condition and to be used once only, in two parts, namely a lid which is merely placed on a base into which the gel nutrient medium is poured after it has been heated to liquefy it.

After the gel medium has cooled and solidified, the Petri dishes may be used or stored in a refrigerator to obtain the optimum storage conditions.

The surface of the gel culture medium is then brought into contact with the microporous filter membrane on the top of which any micro-organisms have been collected and development of the micro-organisms is then dependent on capillary action through the pores of the membrane.

The use of Petri dishes has a certain number of disadvantages.

Firstly, the surface of the gel medium may feature a certain number of defects due either to an inadequate casting temperature or to excessively sudden cooling and the resulting so-called "orange peel" effect results in surface defects which can compromise good contact between the culture medium and the bottom of the filter membrane essential for the micro-organisms to develop.

Also, the surface of the gel medium is deformed as its edge is not flat but rises by capillary action up the vertical walls of the base of the Petri dish, forming what is referred to as a fillet; this limits the surface area of the gel that can be used and filter membranes with a diameter substantially less than that of the Petri dish are frequently used, whence an increase in overall size and an unused quantity of gel.

When the Petri dish is being filled with the gel medium there is also the risk that air bubbles may rise and burst on the surface and, because of the viscous nature of the medium, produce craters compromising good contact with the filter membrane.

Given that Petri dishes are filled at a raised temperature, as the culture medium cools condensation occurs on the bottom of the lid; the condensate sometimes runs down onto the top of the gel medium, requiring a drying operation in an oven before use. This phenomenon is accentuated when the dishes are stored in a refrigerator.

The surface of the gel culture medium is of course exposed to risks of exogenous contamination when it is stored in Petri dishes as these are not sealed.

Finally, Petri dishes do not enable direct counting of any germs present on a surface by simple contact between the surface of the gel medium and said surface, because of its concave shape.

US patent 4 326 028 discloses an improved Petri dish comprising a cylindrical casing to which is attached a perforated support grid and which is intended to receive in back-to-back relationship two different culture mediums to enable observation in two compartments situated one on each side of the grid; however, the surfaces of both of the two culture mediums are subject to the same disadvantages as those that affect Petri dishes.

An object of the present invention is an improved container enabling the same usage as Petri dishes but without the above disadvantages.

This container, intended to receive one or more culture mediums for micro-organisms, and which is preferably in a sterile form for use once only, and which may be made from glass or a plastics material, comprises three parts, namely a cylindrical casing open at both ends and provided with a perforated support grid joined by its circular edge to the inside walls of the casing and a base and a lid.

A container in accordance with the invention is characterized in that the upper end of the casing fits in a liquid-tight way to the lid so that a culture medium at a raised temperature can be cast while the container is resting on its lid.

To fill a container of this kind with a micro-organism culture medium it is turned upside down to rest on its liquid-tight lid, the base is removed and the culture medium is poured into the casing, fitted only with its lid, until the perforated support grid is immersed in the medium; the base is then replaced and the container, which may be stored in a refrigerator for subsequent use, is turned over again so as to rest on its base, with the cover on the top, ready for use.

By virtue of this structure and this filling method, the surface of the nutrient medium which is used for the contact with the micro-organisms is not the top surface resulting from pouring it but, because the container is turned over, the bottom surface which is directly in contact with the lid, to the surface state of which particular attention is given.

The surface of the medium, which is therefore molded according to the shape of the lid, thus does not have any of the defects mentioned hereinabove.

In particular, it will not suffer from the orange peel effect, nor will it have any fillets at its edge and nor will it have any craters due to air bubbles.

There is no risk of condensation of water on or exogenous contamination of the surface of the nutrient medium which will be brought into contact with the micro-organisms to be detected since this surface is in direct contact with the previously sterilized lid.

Finally, by making the lid the required shape, for example a flat or slightly concave shape, it is possible to obtain a nutrient medium surface of corresponding flat or convex shape, which enables it to be applied directly by contact onto a surface to be checked for contamination, an operation which is impossible with an ordinary Petri dish.

The container in accordance with the present invention may be employed with all microbiological test methods utilizing a microporous filter membrane and in particular the process which is the subject matter of French patent No 84 01470 filed 31 January 1984 by the applicant.

The invention will now be described with reference to the appended drawings, in which:

- figure 1 is a view in cross-section of a first embodiment of the container in accordance with the invention, in the position for filling it with the nutrient medium;
- figure 2 is a schematic representation of a second embodiment of the container in accordance with the invention, in the position for filling it with the nutrient medium ;
- figure 3 is a schematic representation of another embodiment of the container in accordance with the invention in a position analogous to that of figure 1;
- figure 4 shows the same embodiment as figure 3 but with the lid turned through 90° relative to the position that it occupied in figure 3;
- figure 5 is a schematic representation of another embodiment of the invention; and
- figure 6 is a plan view of the cylindrical casing for the embodiment shown in figure 5.

The container 1 in accordance with the present invention comprises three component parts, namely a cylindrical casing 2, a lid 3 and a base 4.

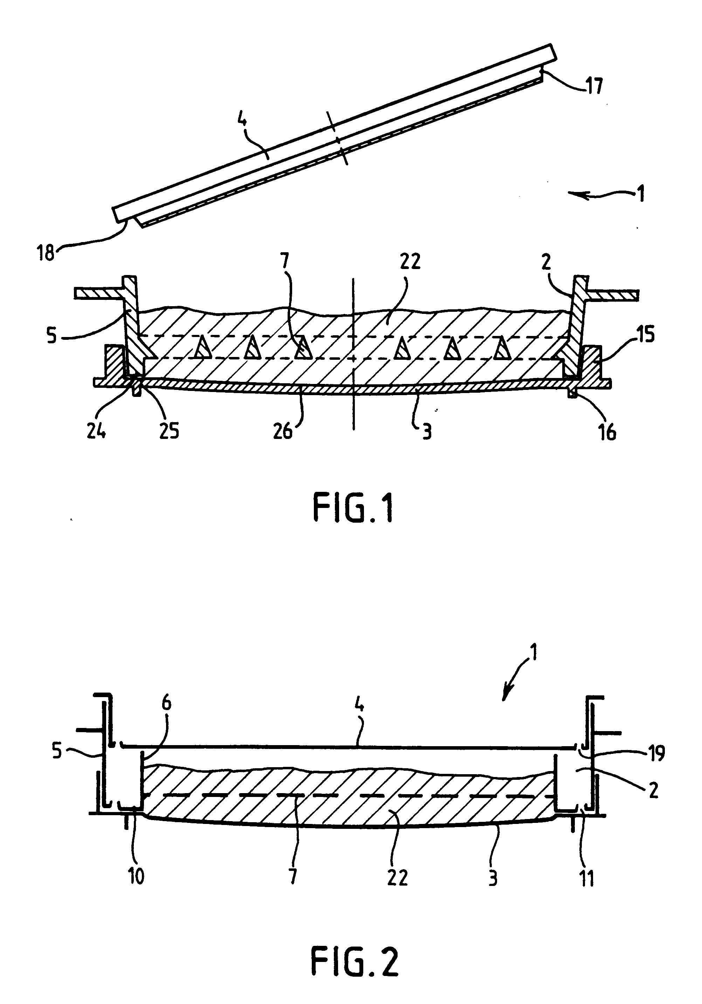

The casing 2 shown in figure 1 comprises a single wall 5 which is slightly frustoconical to enable a liquid-tight fit between the two open ends of the casing and the lid 3 and the base 4, respectively.

Near the upper end of the casing 2 the wall 5 is attached to a perforated support grid 7 designed to support the culture medium and which has a flat circular shape coaxial with the casing 2.

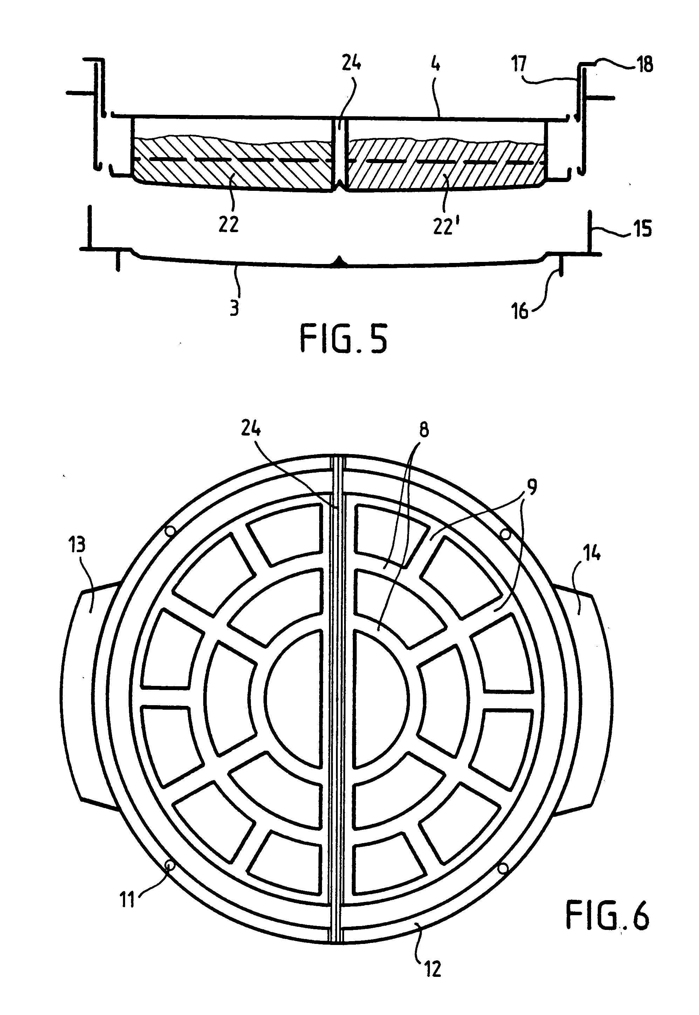

This grid 7, which is attached at its circular edge to the wall 5 of the casing, may be in the form of an array of concentric circular elements 8 and radial elements 9 (see figure 6), preferably having a top surface, that is to say that facing towards the lid 3, that is flat and having a transverse cross-section that may be triangular.

This form of grid retains more readily the gel culture medium when the container is in its position for use like a Petri dish, that is to say upside down relative to that shown in figure 1.

The upper end of the casing 2 has a flat annular surface 24 lying in a plane perpendicular to the axis of the casing 2.

Finally, the casing 2 features handling members, specifically two diametrally opposed handling lugs 13 and 14 (see figure 6).

The lid 3 is in the form of a circular member defining an upper surface the edge of which is in one piece with a low substantially cylindrical rim 15 which is preferably slightly frustoconical to enable it to be nested in a sealed way to the open upper end of the casing 2.

As shown in figures 1 through 5, the lid 3 may be in the form of a circular member defining a flat annulus 25 cooperating with the flat surface 24 of the casing and a central inside surface 26 which is slightly concave on the side facing towards the casing, the convex other side being preferably provided with a cylindrical stand 16 of smaller diameter serving to support the container when it is turned upside down and which compensates for the convex shape of said side.

This concave shape of the lid 3 is of particular benefit in that it enables the surface of the nutrient medium that is to be brought into contact with the micro-organisms to be detected to be molded with a convex shape.

Like the lid 3, the base 4 is in the form of a circular member defining a flat surface the edge of which is in one piece with a small substantially cylindrical rim 17 which is preferably slightly frustoconical to enable it to be nested in a sealed way with the open lower end of the casing 2.

Said rim 17 may incorporate a step 18 to facilitate grasping it to disengage the base 4 from the casing 2.

The lid 3 and the base 4 may each comprise handling members to facilitate nesting them in a sealed way onto the casing 2.

The embodiment shown schematically in figure 2 differs from that shown in figure 1 in that the casing 2 comprises two concentric and substantially cylindrical walls 5 and 6 instead of a single wall 5.

In this second embodiment, which is specifically designed for use with the sleeve which is the subject matter of the previously mentioned French patent No 84 01470, the outer wall 5 is slightly frustoconical to enable, as in the figure 1 embodiment, sealed nesting of the two open ends of the casing to the lid 3 and the base 4, respectively.

The inside wall 6 is in one piece with the perforated support grid 7.

And the two concentric walls 5 and 6 are joined at their upper end by a flat joining annulus 10 coaxial with the casing. This annulus 10 comprises a certain number of axial perforations 11, four as shown in figure 6, for example, preferably formed in a circular groove 12 situated on the upper surface of the annulus 10.

In this second embodiment the base 4 comprises near its edge and inside the rim 17 axial perforations 19 serving as vents and preferably situated on the same vertical line as the annulus 10 joining the two concentric walls 5 and 6 of the casing to provide communication between the volume defined by these two walls and the external environment and thus to prevent any piston effect between the base 4 and the casing 2 of the container in accordance with the invention.

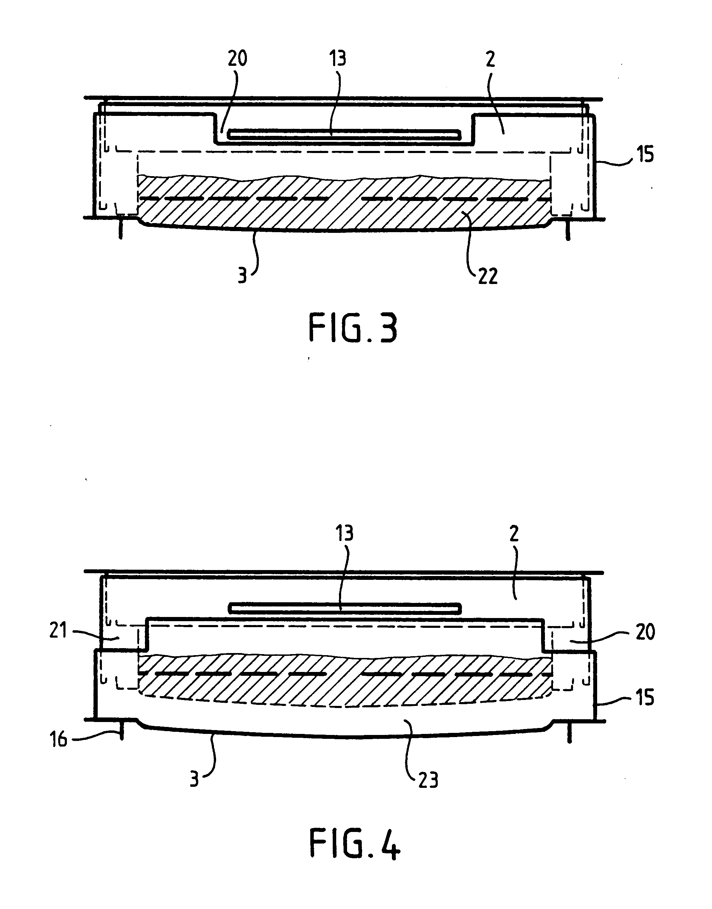

A third embodiment shown in figures 3 and 4 differs from that shown in figure 1 or 2 in terms of the shape of the lid 3.

In figures 3 and 4 the lid 3 has a rim 15 which is substantially longer in the axial direction and which includes two diametrally opposed notches 20 and 21 on the side opposite the support 16.

These notches 20 and 21 in the lid cooperate with the holding lugs 13 and 14 on the casing to hold these two parts nested one within the other either to the maximum depth, in which position the culture medium 22 is in contact with the lid 3 as shown in figure 3, or, following simple rotation of the lid relative to the casing through 90°, to hold these two parts of the container nested one within the other to a lesser depth, leaving an incubation chamber 23 between the culture medium and the lid, as shown in figure 4.

A fourth embodiment shown in figures 5 and 6 differs from that shown in the previous figures in that the casing 4 comprises a sealed barrier 24, which makes it possible to introduce two different culture mediums 22 and 22′.

It is obvious that the container in accordance with the present invention, given the advantages that it has, is particularly beneficial in numerous applications. It is usable not only as a Petri dish but also for checking microbiological contamination of surfaces; it is also suitable for the biological testing method and device using a sleeve which is the subject matter of the previously mentioned French patent No 84 01470; finally, it is able to contain one or more culture mediums to provide for counting different micro-organisms captured on the same microporous filter membrane, which considerably reduces the testing time, the amount of equipment to be used, and consequently the cost of the test.

**Claims**

1. A container (1) intended to receive one or more culture mediums (22 and 22') for micro-organisms, preferably in sterile form for use once only comprising a casing (2) open at both ends and provided, in a transverse section, with a perforated support grid (7), joined by its circular edge to the inside wall of the casing (2), and a lid (3) and a base (4), characterized in that the casing (2) has a slightly frusto-conical shape and the base (4) and the lid (3) have corresponding slightly frusto-conical shapes enabling them to be fitted in a sealed way to each of the two open ends of the casing (2), so as to enable hot casting of a culture medium while the container is resting on its lid.

2. Container according to claim 1, characterized in that the upper end of the casing (2) has a flat annular surface (24) situated in a plane perpendicular to the axis of the casing and which cooperates with a corresponding flat surface (25) on the lid (3) to secure the sealed fit of the lid to this end of the casing.

3. Container according to any one of the preceding claims, characterized in that the lid (3) is in the form of a circular member the edge of which is is one piece with a slightly frusto-conical rim (15), said circular member defining a flat annulus cooperating in a sealed way with the upper end of the casing (2) and a central inside surface which is optionally slightly concave on the side that faces towards the casing (2), said member preferably being provided on the other side with a cylindrical stand (16) of smaller diameter serving as a support when the container (1) rests on its lid (3).

4. Container according to any one of the preceding claims, characterized in that the base (4) is in the form of a circular member defining a flat surface and the edge of which is in one piece with a slightly frusto-conical rim (17), said rim (17) optionally having a step (18) limiting nesting of the base (4) onto the casing (2).

5. Container according to any one of the preceding claims, characterized in that the casing (2) comprises two concentric and substantially cylindrical walls (5 and 6) of which the outer wall (5) is slightly frusto-conical and of which the inner wall (6) is in one piece with the perforated support grid (7), these two walls (5 and 6) being joined at their upper edges by a flat joining annulus (10) comprising a certain number of axial perforations (11) preferably formed in a circular groove (12) situated on the upper side of the annulus (10).

6. Container according to any one of the preceding claims, characterized in that the casing (2), the base (4), the lid (3) or any two thereof or all three comprise handling members.

7. Container according to any one of the preceding claims, characterized in that the perforated support grid (7) from the casing (2) is in the form of an array of concentric circular elements (8) and radial elements (9) preferably having a flat upper surface and the cross-section of which may be triangular.

8. Container according to the preceding claims 3 and 6 characterized in that the casing (2) has diametrally opposed handling lugs (13 and 14) and in that the rim (15) of the cover (3) has two diametrally opposed notches (20 and 21) adapted to receive the handling lugs (13 and 14) of the casing in two positions situated at different depths by rotation through 90° of the cover (3) relative to said casing (4).

9. Method of filling with one or more culture mediums for micro-organisms the container (1) according to any one of claims 1 through 8, characterized in that the culture medium or mediums (22 and 22') is or are poured into the container (1) while the latter is resting on its lid (3), the base (4) having been removed beforehand.

10. Application of the container according to any one of claims 1 through 8 to the detection of micro-organisms characterized in that, after filling said container by the method according to claim 10 and nesting the base (4) onto the casing (2) the container rests on the member serving it as a base (4) and the lid (3) is removed to use the surface of the culture medium which was in contact with the lid (3) to receive micro-organisms whose presence is to be detected.

**Revendications**

1. Récipient (1) destiné à recevoir un ou plusieurs milieux de culture (22 et 22') pour des microorganismes se présentant de préférence sous forme stérile à usage unique, comportant un boîtier (2) ouvert aux deux extrémités et muni, selon une section transversale, d'une grille de support perforée (7) reliée par sa périphérie circulaire à la paroi interne du boîtier (2), et un couvercle (3) et un fond (4), caractérisé en ce que le boîtier (2) présente une forme légèrement tronconique et le fond (4) et le couvercle (3) ont des formes légèrement tronconiques correspondantes leur permettant d'être ajustés de manière étanche sur chacune des deux extrémités ouvertes du boîtier (2) afin de permettre la coulée à chaud d'un milieu de culture alors que le récipient repose sur son couvercle.

2. Récipient selon la revendication 1, caractérisé en ce que l'extrémité supérieure du boîtier (2) présente une surface plane (24) en forme de couronne située dans un plan perpendiculaire à l'axe du boîtier et qui coopère avec une surface plane correspondante (25) du couvercle (3) pour assurer l'ajustement étanche du couvercle sur cette extrémité du boîtier.

3. Récipient selon l'une des revendications précédentes, caractérisé en ce que le couvercle (3) se présente sous la forme d'un élément circulaire dont la périphérie est d'une seule pièce avec un rebord (15) légèrement tronconique, ledit élément circulaire définissant une couronne plane coopérant de manière étanche avec l'extrémité supérieure du boîtier (2) et une surface intérieure centrale qui peut être légèrement concave du côté tourné vers le boîtier (2), ledit élément étant muni de préférence de l'autre côté d'un pied cylindrique (16) de plus faible diamètre servant de support lorsque le récipient (1) repose sur son couvercle (3).

4. Récipient selon l'une quelconque des revendications précédentes, caractérisé en ce que le fond (4) se présente sous la forme d'un élément circulaire définissant une surface plane et dont la périphérie est d'une seule pièce avec un rebord légèrement tronconique (17), ledit rebord (17) présentant éventuellement un gradin (18) limitant l'emboîtement du fond (4) sur le boîtier (2).

5. Récipient selon l'une quelconque des revendications précédentes, caractérisé en ce que le boîtier (2) comporte deux parois sensiblement cylindriques et concentriques (5 et 6) dont la paroi externe (5) est légèrement tronconique et dont la paroi interne (6) est d'une seule pièce avec la grille de support perforée (7), ces deux parois (5 et 6) étant reliées à leurs extrémités supérieures par une couronne circulaire plane de raccordement (10) présentant un certain nombre de perforations axiales (11) pratiquées de préférence dans une gorge circulaire (12) située sur la face supérieure de la couronne (10).

6. Récipient selon l'une quelconque des revendications précédentes, caractérisé en ce que le boîtier (2), le fond (4), le couvercle (3) ou deux quelconques d'entre eux ou les trois, comprennent des éléments de préhension.

7. Récipient selon l'une quelconque des revendications précédentes, caractérisé en ce que la grille de support perforée (7) du boîtier (2) se présente sous la forme d'un réseau d'éléments circulaires concentriques (8) et d'éléments radiaux (9) présentant de préférence une surface supérieure plane et dont la section peut être triangulaire.

8. Récipient selon les revendications précédentes 3 et 6, caractérisé en ce que le boîtier (2) présente des oreilles de préhension (13 et 14) diamétralement opposées et en ce que le rebord (15) du couvercle (3) présente deux échancrures diamétralement opposées (20 et 21) conçues pour recevoir les oreilles de préhension (13 et 14) du boîtier dans deux positions situées à des profondeurs différentes par rotation de 90° du couvercle (3) par rapport audit boîtier (4).

9. Procédé de remplissage avec un ou plusieurs milieux de culture pour micro-organismes du récipient (1) selon l'une quelconque des revendications 1 à 8, caractérisé en ce que l'on verse le milieu ou les milieux de culture (22 et 22') dans le récipient (1) tandis que celui-ci repose sur son couvercle (3), le fond (4) ayant été préalablement enlevé.

10. Application du récipient selon l'une quelconque des revendications 1 à 8 à la détection de micro-organismes, caractérisée en ce que, après remplissage dudit récipient par le procédé selon la revendication 10, et emboîtement du fond (4) sur le boîtier (2), le récipient repose sur l'élément lui servant de fond (4) et on enlève le couvercle (3) pour utiliser la surface du milieu de culture qui était en contact avec le couvercle (3) pour recevoir les micro-organismes dont on doit détecter la présence.

**Patentansprüche**

1. Behälter (1) zur Aufnahme eines oder mehrerer Kulturmedien (22 und 22') für Mikroorganismen, bevorzugt in steriler Ausführung zum Einmalgebrauch, mit einem an beiden Enden offenen Gehäuse (2), das in einem Querschnitt einen mit Durchbrechungen versehenen Stützrost (7) aufweist, der mit seinem Umlaufrand an der Innenwand des Gehäuses (2) befestigt ist, sowie mit einem Deckel (3) und mit einem Boden (4), **dadurch gekennzeichnet,** daß das Gehäuse (2) eine leicht kegelstumpfförmige Form aufweist und daß der Boden (4) und der Deckel (3) mit entsprechenden leicht kegelstumpfförmigen Formen ausgebildet sind, die es ermöglichen, sie dichtend jeweils an einem der beiden offenen Enden des Gehäuses (2) derart anzubringen, daß ein heißes Eingießen eines Kulturmediums erfolgen kann, während der Behälter auf seinem Deckel steht.

2. Behälter nach Anspruch 1, dadurch gekennzeichnet, daß das obere Ende des Gehäuses (2) eine ebene Ringfläche (24) aufweist, die in einer Ebene senkrecht zur Gehäuseachse liegt und mit einer entsprechenden ebenen Fläche (25) am Deckel (3) zusammenwirkt, um den dichten Sitz des Deckels auf diesem Gehäuseende zu gewährleisten.

3. Behälter nach einem der vorhergehenden Ansprüche, dadurch gekennzeichnet, daß der Deckel (3) in Form eines kreisförmigen Teiles ausgebildet ist, dessen Rand einstückig mit einer leicht kegelstumpfförmigen Randfassung (15) ausgeführt ist, wobei das kreisförmige Teil eine ebene Ringfläche, die dichtend mit dem oberen Ende des Gehäuses (2) zusammenwirkt, und eine innenliegende Mittelfläche ausbildet, die auf ihrer dem Gehäuse (2) zugewendeten Seite wahlweise leicht konkav geformt ist, und wobei bevorzugt das kreisförmige Teil auf seiner anderen Seite mit einem zylindrischen Ständer (16) kleineren Durchmessers versehen ist, der als Stütze dient, wenn der Behälter (1) auf seinem Deckel (3) steht.

4. Behälter noch einem der vorhergehenden Ansprüche, dadurch gekennzeichnet, daß der Boden (4) in Form eines kreisförmigen Teiles ausgeführt ist, das eine ebene Fläche bildet und dessen Rand einstückig mit einer leicht kegelstumpfförmigen Randfassung (17) ausgebildet ist, die wahlweise mit einer Stufe (18) als Begrenzung beim Aufstecken des Bodens (4) auf das Gehäuse (2) versehen ist.

5. Behälter nach einem der vorhergehenden Ansprüche, dadurch gekennzeichnet, daß das Gehäuse (2) zwei konzentrische und im wesentlichen zylindrische Wände (5 und 6) aufweist, deren äußere (5) leicht kegelstumpfförmig und deren innere (6) einstückig mit dem mit Durchbrechungen versehenen Stützrost (7) ausgebildet ist, und daß beide Wände (5 und 6) an ihren oberen Rändern durch einen ebenen Verbindungsring (10) miteinander verbunden sind, der eine bestimmte Anzahl von axialen Öffnungen (11) aufweist, die bevorzugt in einer Ringnut (12) auf der Oberseite des Verbindungsrings (10) angebracht sind.

6. Behälter nach einem der vorhergehenden Ansprüche, dadurch gekennzeichnet, daß das Gehäuse (2), der Boden (4), der Deckel (3) oder zwei dieser Elemente oder alle drei Elemente mit Teilen versehen sind, die zu ihrer Handhabung dienen.

7. Behälter nach einem der vorhergehenden Ansprüche, dadurch gekennzeichnet, daß der mit Durchbrechungen versehene Stützrost (7) des Gehäuses (2) in Form einer Anordnung von konzentrischen, kreisförmigen Elementen (8) und von radial verlaufenden Elementen (9) ausgebildet ist, die vorzugsweise eine ebene Oberfläche aufweisen und deren Querschnitt dreieckförmig ausgebildet sein kann.

8. Behälter nach den vorhergegangenen Ansprüchen 3 und 6, dadurch gekennzeichnet, daß das Gehäuse (2) mit einander diametral gegenüberliegenden Haltegriffen (13 und 14) versehen ist und die Randfassung (15) des Dekkel (3) zwei einander diametral gegenüberliegende Ausnehmungen (20 und 21) aufweist, die zur Aufnahme der Haltegriffe (13 und 14) des Gehäuses (2) in zwei unterschiedlichen Höhenlagen bei einer Drehung des Deckels (3) um 90° relativ zum Gehäuse (2) dienen.

9. Verfahren zur Befüllung des Behälters (1) nach einem der Ansprüche 1 bis 8 mit einem oder mehreren Kulturmedien für Mikroorganismen, dadurch gekennzeichnet, daß das Kulturmedium oder die Kulturmedien (22 und 22') in den Behälter (1) eingegossen wird/werden, während dieser auf seinem Deckel (3) steht und zuvor sein Boden (4) entfernt wurde.

10. Anwendung des Behälters nach einem der Ansprüche 1 bis 8 zur Feststellung von Mikroorganismen, dadurch gekennzeichnet, daß nach dem Befüllen des Behälters gemäß dem Verfahren nach Anspruch 9 und dem Aufstecken des Bodens (4) auf das Gehäuse (2) der Behälter auf dem Teil steht, das ihm als Boden (4) dient, und daß der Deckel (3) entfernt wird, um die Oberfläche des Kulturmediums, das sich in Kontakt mit dem Deckel (3) befand, zur Aufnahme von Mikroorganismen, deren Vorhandensein festgestellt werden soll, zu benützen.

EP 0 267 093 B1

FIG.1

FIG.2

FIG.3

FIG.4

FIG.5

FIG.6